# EUROPEAN PATENT SPECIFICATION

(11) **EP 1 294 673 B1**
(45) Date of publication and mention of the grant of the patent: **16.03.2005**
(21) Application number: 01945647.4
(22) Date of filing: 27.06.2001
(51) Int. Cl.: C07C 67/04, B01J 27/188

(54) **CATALYST FOR USE IN PRODUCING LOWER ALIPHATIC CARBOXYLIC ACID ESTER, PROCESS FOR PRODUCING THE CATALYST AND PROCESS FOR PRODUCING LOWER ALIPHATIC CARBOXYLIC ACID ESTER USING THE CATALYST**
KATALYSATOR BRAUCHBAR FÜR DIE HERSTELLUNG VON NIEDRIGEM ALIPHATISCHEM CARBONSÄUREESTER,VERFAHREN FÜR DIE HERSTELLUNG DES KATALYSATORS UND VERFAHREN FÜR DIE HERSTELLUNG DER NIEDRIGEN ALIPHATISCHEN CARBONSÄUREESSTER UNTER VERWENDUNG DES KATALYSATORS
CATALYSEUR UTILISE DANS LA PRODUCTION D'UN ESTER D'ACIDE CARBOXYLIQUE ALIPHATIQUE INFERIEUR, PROCEDE DE PRODUCTION DU CATALYSEUR ET PROCEDE DE PRODUCTION D'UN ESTER D'ACIDE CARBOXYLIQUE ALIPHATIQUE INFERIEUR A L'AIDE DU CATALYSEUR

(30) Priority: 27.06.2000 JP 2000192964; 18.07.2000 US 218803 P
(43) Date of publication of application: 26.03.2003
(73) Proprietor: Showa Denko K.K., Tokyo 105-8518 (JP)
(72) Inventor: KADOWAKI, Etsuko, c/o Oita Plant, Showa Denko K K, Oita-shi, Oita 870-0189 (JP); HIGASHI, Tomoyoshi, c/o Oita Plant, Showa Denko KK, Oita-shi, Oita 870-0189 (JP); OGUCHI, Wataru, C/O Oita Plant, Showa Denko K K, Oita-shi, Oita 870-0189 (JP); UCHIDA, Hiroshi,c/o Oita Plant, Showa Denko KK, Oita-shi, Oita 870-0189 (JP)
(74) Representative: Strehl Schübel-Hopf & Partner
(86) International application number: PCT/JP2001/005532
(87) International publication number: WO 2002/000589

(56) References cited:
- EP-A- 0 538 826
- EP-A- 0 562 139
- EP-A- 0 757 027
- EP-A- 0 936 210
- EP-A- 0 959 064
- WO-A-00/03966
- WO-A-00/74842
- DATABASE WPI Section Ch, Week 200014 Derwent Publications Ltd., London, GB; Class E17, AN 1993-252691 XP002189514 & JP 03 012059 B (SHOWA DENKO KK), 21 February 2000 (2000-02-21)

## Description

### Technical Field

The present invention relates to a catalyst for use in producing a lower aliphatic carboxylic acid ester from a lower olefin and a lower aliphatic carboxylic acid, a process for producing the catalyst, and a process for producing a lower aliphatic carboxylic acid ester using the catalyst.

More specifically, the present invention relates to a catalyst for use in producing a lower aliphatic carboxylic acid ester, which contains a specific heteropolyacid salt and which is used in the process for producing a lower aliphatic carboxylic acid ester by esterifying a lower aliphatic carboxylic acid with a lower olefin, wherein the catalyst is contacted with a gas containing at least one member selected from the group consisting of water, lower aliphatic carboxylic acids and lower aliphatic alcohols before the reaction; a process for producing the catalyst; and a process for producing a lower aliphatic carboxylic acid ester using the catalyst.

### Background Art

It is well known that a corresponding ester can be produced from a lower aliphatic carboxylic acid and an olefin. Also, a catalyst comprising a heteropolyacid and/or a salt thereof is known to effectively act in this reaction. Specific examples of such a catalyst include those described in Japanese Unexamined Patent Publications No. 4-139148 (JP-A-4-139148), No. 4-13949 (JP-A-4-139149), No. 5-65248 (JP-A-5-65248) and No. 5-294894 (JP-A-5-294894).

Among these specific examples, the catalysts containing at least one salt selected from the group consisting of cesium salts, rubidium salts, thallium salts, ammonium salts and potassium salts of phosphotungstic acid, silicotungstic acid, phosphomolybdic acid and silicomolybdic acid disclosed in JP-A-4-139148, JP-A-4-139149 and JP-A-5-65248, exhibit high initial activity and relatively high space time yield. However, in use on an industrial base, for example, the activity decreases and the space time yield also lowers with the progress of reaction and this was a problem to be solved.

In order to solve this problem, Japanese Unexamined Patent Publication No. 5-170698 (JP-A-5-170698) proposes a method of contacting a catalyst described above with water and an organic carboxylic acid or an organic carboxylic acid ester in a gas phase before performing the reaction. In a production process of a lower aliphatic carboxylic acid ester using a catalyst subjected to a pre-contacting treatment according to the above-described method, it is revealed that the catalyst exhibits high initial activity and the activity scarcely decreases as compared with conventional production processes using a catalyst which is not subjected to the contacting treatment.

On the other hand, Japanese Unexamined Patent Publication No. 5-294894 (JP-A-5-294894) discloses a catalyst obtained by loading at least one heteropolyacid salt selected from the group consisting of lithium salts, copper salts, magnesium salts and gallium salts of heteropolyacids on a support and it is revealed that although the method disclosed in JP-A-5-170698 of contacting a catalyst with water and an organic carboxylic acid or an organic carboxylic acid ester in a gas phase before performing the reaction is not used, the catalyst can exhibit initial activity equal thereto or higher than that and can maintain the activity.

In other words, with respect to the initial activity and space time yield of the catalyst and the maintenance thereof (so-called catalyst life), the problems have been overcome by the catalyst disclosed in JP-A-5-294894. The matter in need of improvement is the production of by-products such as olefins, having 3 or more carbon atoms including butene, and aldehydes.

The production of these by-products directly causes reduction in the selectivity of reaction. Furthermore, these by-products sometimes become catalyst poisons in the reaction of a lower olefin with a lower aliphatic carboxylic acid and may conspicuously inhibit the reaction.

Particularly, in industrially performing the process for producing a lower alphatic carboxylic acid ester through the reaction, a circulation process is generally employed mainly for recycling unreacted raw materials so as to increase the reaction efficiency in view of profitability. In this case, if those by-products are not removed and returned to a reaction system via a circulation system, the catalyst may be damaged and the catalyst life greatly shortened.

To solve this problem, the methods described, for example, in Japanese Unexamined Patent Publications No. 11-269126 (JP-A-11-269126) and No. 11-335323 (JP-A-11-335323) may be used. JP-A-11-269126 discloses a method of removing acetaldehyde impurities at the reactor inlet by distillation or by reacting them with a compound highly reactive with an aldehyde group. Also, JP-A-11-335323 discloses a method of newly providing a step for removing acetaldehyde using an acetaldehyde-removing column and thereby separating it. In either case, however, a new separation step must be provided, therefore, the process becomes very cumbersome and the costs increase.

As such, it has been proposed to provide a step for separating and removing by-products so that the catalyst life can be maintained even in the process employing a circulation system. However, means to solve this problem from the standpoint of sufficiently suppressing the production of by-products themselves in the reaction has not yet been found.

### Disclosure of Invention

The object of the present invention is to provide a catalyst for use in producing a lower aliphatic carboxylic acid ester, which is used in producing a lower aliphatic carboxylic acid ester from a lower olefin and a lower aliphatic carboxylic acid and which is a catalyst having high initial activity and high space time yield, favored with a catalyst life long enough to endure practical use in industry and capable of reducing the production of by-products; a process for producing the catalyst; and a process for producing a lower aliphatic carboxylic acid ester using the catalyst.

The present inventors have made extensive investigations into a catalyst not only capable of exhibiting high initial activity and sufficiently long catalyst life at the reaction of a lower olefin and a lower aliphatic carboxylic acid in a gas phase to produce a lower fatty acid ester, but also reduced in the production of by-products represented by butene and aldehydes which work out to a catalyst poison. As a result, it has been found that when a specific step is provided in the preparation of a catalyst, the catalyst can be greatly reduced in the production of by-products harmful to the catalyst, such as butene and aldehydes. The present invention has been accomplished based on this finding.

More specifically, the present invention (I) is a catalyst for use in producing a lower aliphatic carboxylic acid ester, which is used in reacting a lower olefin with a lower aliphatic carboxylic acid in a gas phase to produce a lower aliphatic carboxylic acid ester, wherein the catalyst is produced by a process comprising the following first and second steps:

### First Step

a step for loading one or more heteropolyacid salts on a support to obtain a heteropolyacid salt supported catalyst; and

### Second Step

a step for contacting the heteropolyacid salt supported catalyst obtained in the first step with a gas containing at least one member selected from the group consisting of water, lower aliphatic carboxylic acids and lower aliphatic alcohols without any olefin to obtain the catalyst for use in producing a lower aliphatic carboxylic acid ester.

The present invention (II) is a process for producing a catalyst for use in producing a lower aliphatic carboxylic acid ester, the catalyst being used in reacting a lower olefin with a lower aliphatic carboxylic acid in a gas phase to produce a lower aliphatic carboxylic acid ester, which process comprises the following first and second steps:

### First Step

a step for loading one or more heteropolyacid salts on a support to obtain a heteropolyacid salt supported catalyst; and

### Second Step

a step for contacting the heteropolyacid salt supported catalyst obtained in the first step with a gas containing at least one member selected from the group consisting of water, lower aliphatic carboxylic acids and lower aliphatic alcohols without any olefin to obtain the catalyst for use in producing a lower aliphatic carboxylic acid ester.

The present invention (III) is a process for producing a lower aliphatic carboxylic acid ester, comprising reacting a lower olefin with a lower aliphatic carboxylic acid in a gas phase in the presence of the catalyst for use in producing a lower aliphatic carboxylic acid ester of the present invention (I).

The present invention (IV) is a process for producing a lower aliphatic carboxylic acid ester comprising reacting a lower olefin with a lower aliphatic carboxylic acid in a gas phase, which process comprises the following first to fourth steps:

### First Step

a step for loading one or more heteropolyacid salts on a support to obtain a heteropolyacid salt supported catalyst;

### Second Step

a step of filling the heteropolyacid salt supported catalyst obtained in the first step, into a reactor for use in the reaction of the lower olefin with the lower aliphatic carboxylic acid in a gas phase;

### Third Step

a step of contacting the heteropolyacid salt supported catalyst filled in the reactor, with a gas containing at least one member selected from the group consisting of water, lower aliphatic carboxylic acids and lower aliphatic alcohols without any olefin; and, distinguished from the Third Step,

### as a Fourth Step

a step of passing a mixed gas containing the lower olefin and the lower aliphatic carboxylic acid through the heteropolyacid salt supported catalyst after the third step, to obtain the lower aliphatic carboxylic acid ester.

### Best Mode for Carrying Out the Invention

The present invention will be described below with reference to preferred embodiments thereof.

The present invention (I) is a catalyst for use in producing a lower aliphatic carboxylic acid ester, which is used in reacting a lower olefin with a lower aliphatic carboxylic acid in a gas phase to produce a lower aliphatic carboxylic acid ester, wherein the catalyst is produced by a process comprising the following first and second steps:

### First Step

a step for loading one or more heteropolyacid salts on a support to obtain a heteropolyacid salt supported catalyst; and

### Second Step

a step for contacting the heteropolyacid salt supported catalyst obtained in the first step with a gas containing at least one member selected from the group consisting of water, lower aliphatic carboxylic acids and lower aliphatic alcohols without any olefin to obtain the catalyst for use in producing a lower aliphatic carboxylic acid ester.

That is, the catalyst for use in producing a lower aliphatic carboxylic acid ester of the present invention (I) is a catalyst obtained by a production process comprising the above-described first and second steps.

The first step is described below.

In the catalyst for use in producing a lower aliphatic carboxylic acid ester of the present invention (I), the heteropolyacid used in the first step comprises a center element and a peripheral element to which oxygen is bonded. The center element is usually silicon or phosphorus but may comprise an optional element selected from various kinds of elements belonging to Groups I to XVII of the Periodic Table.

Specific examples of the center element include cupric ion; divalent beryllium, zinc, cobalt and nickel ions; trivalent boron, aluminum, gallium, iron, cerium, arsenic, antimony, phosphorus, bismuth, chromium and rhodium ions; tetravalent silicon, germanium, tin, titanium, zirconium, vanadium, sulfur, tellurium, manganese, nickel, platinum, thorium, hafnium and cerium ions and other rare earth ions; pentavalent phosphorus, arsenic, vanadium and antimony ions; hexavalent tellurium ion; and heptavalent iodide ion, however, the present invention is by no means limited thereto. Specific examples of the peripheral element include tungsten, molybdenum, vanadium, niobium and tantalum, however, the present invention is by no means limited thereto.

These heteropolyacids are also known as a "polyoxoanion", a "polyoxometallic salt" or a "metal oxide cluster". Some well known structures of anions are named after a researcher himself in this field, for example, as Keggin, Wells-Dawson or Anderson-Evans-Perloff structures. There are detailed description in "Porisan no Kagaku, Kikan Kagaku Sousetau (Chemistry of Polyacids, Seasonal Publication for Introduction to Chemistry)", No. 20, 1993, edited by Japan Chemical Society. Heteropolyacids usually have a high molecular weighty for example, a molecular weight of 700 to 8,500, and include dimeric complexes.

Specific examples of the heteropolyacid which can be used as the starting material of heteropolyacid salt in the catalyst for use in producing a lower aliphatic carboxylic acid ester of the present invention (I) include :

| | |
|---|---|
| Tungstosilicic acid | H₄[SiW₁₂O₄₀]·xH₂O |
| Tungstophosphoric acid | H₃[PW₁₂O₄₀]·xH₂O |
| Molybdophosphoric acid | H₃[PMo₁₂O₄₀]·xH₂O |
| Molybdosilicic acid | H₄[SiMo₁₂O₄₀]·xH₂O |
| Vanadotungstosilicic acid | H₄₊ₙ[SiVₙW₁₂₋ₙO₄₀]·xH₂O. |
| Vanadotungstophosphoric acid | H₃₊ₙ[PVₙW₁₂₋ₙO₄₀]·xH₂O. |
| Vanadomolybdophosphoric acid | H₃₊ₙ[PVₙMo₁₂₋ₙO₄₀]·xH₂O |
| Vanadomolybdosilicic acid | H₄₊ₙ[SiVₙMo₁₂₋ₙO₄₀]·xH₂O. |
| Molybdotungstosilicic acid | H₄[SiMoₙW₁₂₋ₙO₄₀]·xH₂O. |
| Mplybdotungstophosphoric acid | H₃[PMOₙW₁₂₋ₙO₄₀]·xH₂O. |

wherein n is an integer of 1 to 11 and x is an integer of at least 1. However, the present invention is by no means limited thereto.

Among these, preferred are tungstosilicic acid, tungstophosphoric acid, molybdophosphoric acid, molybdosilicic acid, vanadotungstosilicic acid and vanadotungstophosphoric acid, and more preferred are tungstosilicic acid, tungstophosphoric acid, vanadotungstosilicic acid and vanadotungstophosphoric acid.

The method of the production of the heteropolyacids are not particularly limited and any methods can be used. For example, they may be produced by heating an acidic aqueous solution (a pH of about 1-2) containing a salt of molybdec acid or tungstic acid and a simple oxygen acid of a hetero-atom or a salt thereof. A heteropolyacid compound may be isolated from the resulting aqueous heteropolyacid solution by a method of separation through precipitation as a metal salt, for example. Specific examples are described in "Shin Jikken Kagaku Kouza 8, Muki Kagoubutsu no Gousei (III) (New Experimental Chemistry Course 8, Synthesis of Inorganic Compounds)", edited by Japan Chemical Society, published by Maruzen K.K., August 20, 1984, Third Edition, page 1413, but the present invention is by no means limited thereto. The Kiggin structure of the obtained heteropolyacid can be confirmed by chemical analysis as well as X-ray diffraction, UV and IR measurements.

The heteropolyacid salt as used in the catalyst for use in producing a lower aliphatic carboxylic acid ester of the present invention (I) is not particularly limited as long as it is a metal salt or an onium salt in which a part or all of hydrogen atoms of the above-described heteropolyacids are substituted. Specific examples thereof include salts of metals such as lithium, sodium, magnesium, barium, copper, gold and gallium, and onium salts, however, the present invention is not limited thereto. Among these, preferred are lithium salts, sodium salts, gallium salts, copper salts and gold salts, more preferred are lithium salts, sodium salts and copper salts.

Heteropolyacids have a relatively high solubility in a polar solvent such as water and other oxygen-containing solvents particularly when the heteropolyacids are free acids or comprise several salts, and the solubility can be controlled by appropriately selecting the counter ion.

Examples of the starting material for the metal element or onium moiety of forming the heteropolyacid salt in the present invention include lithium nitrate, lithium acetate, lithium sulfate, lithium sulfite, lithium carbonate, lithium phosphate, lithium oxalate, lithium nitrite, lithium chloride, lithium citrate, sodium nitrate, sodium acetate, sodium sulfate, sodium carbonate, monosodium phosphate, disodium phosphate, sodium oxalate, sodium nitrite, sodium chloride, sodium citrate, magnesium nitrate hexahydrate, magnesium acetate tetrahydrate, magnesium sulfate, magnesium carbonate, magnesium phosphate tricosahydrate, magnesium oxalate dihydrate, magnesium chloride, magnesium citrate, barium nitrate, barium acetate, barium sulfate, barium carbonate, barium hydrogenphosphate, barium oxalate monohydrate, barium sulfite, barium chloride, barium citrate, copper nitrate, copper acetate, copper sulfate, copper carbonate, copper diphosphate, copper oxalate, copper chloride, copper citrate, aurous chloride, chloroauric acid, auric oxide, auric hydroxide, auric sulfide, aurous sulfide, gallium dichloride, gallium monochloride, gallium citrate, gallium acetate, gallium nitrate, gallium sulfate, gallium phosphate, ammonium acetate, ammonium carbonate, ammonium nitrate, ammonium dihydrogenphosphate, ammonium hydrogencarbonate, ammonium citrate, ammonium nitrate, diammonium phosphate, monoammonium phosphate and ammonium sulfate, however, the present invention is by no means limited thereto.

Among these, preferred are lithium nitrate, lithium acetate, lithium carbonate, lithium oxalate, lithium citrate, sodium nitrate, sodium acetate, sodium carbonate, sodium oxalate, sodium citrate, copper nitrate, copper acetate, copper carbonate, copper citrate, aurous chloride, chloroauric acid, gallium citrate, gallium acetate and gallium nitrate, and more preferred are lithium nitrate, lithium acetate, lithium carbonate, lithium oxalate, lithium citrate, sodium nitrate, sodium acetate, sodium carbonate, sodium oxalate, sodium citrate, copper nitrate, copper acetate, copper carbonate and copper citrate.

Specific examples of the heteropolyacid salt which can be used in the catalyst for use in producing a lower aliphatic carboxylic acid ester of the present invention (I) include lithium salt of tungstosilicic acid, sodium salt of tungstosilicic acid, copper salt of tungstosilicic acid, gold salt of tungstosilicic acid, gallium salt of tungstosilicic acid, lithium salt of tungstophosphoric acid, sodium salt of tungstophosphoric acid, copper salt of tungstophosphoric acid, gold salt of tungstophosphoric acid, gallium salt of tungstophosphoric acid, lithium salt of molybdophosphoric acid, sodium salt of molybdophosphoric acid, copper salt of molybdophosphoric acid, gold salt of molybdophosphoric acid, gallium salt of molybdophosphoric acid, lithium salt of molybdosilicic acid, sodium salt of molybdosilicic acid, copper salt of molybdosilicic acid, gold salt of molybdosilicic acid, gallium salt of molybdosilicic acid, lithium salt of vanadotungstosilicic acid, sodium salt of vanadotungstosilicic acid, copper salt of vanadotungstosilicic acid, gold salt of vanadotungstosilicic acid, gallium salt of vanadotungstosilicic acid, lithium salt of vanadotungstophosphoric acid, sodium salt of vanadotungstophosphoric acid, copper salt of vanadotungstophosphoric acid, gold salt of vanadotungstophosphoric acid, gallium salt of vanadotungstophosphoric acid, lithium salt of vanadomolybdophosphoric acid, sodium salt of vanadomolybdophosphoric acid, copper salt of vanadomolybdophosphoric acid, gold salt of vanadomolybdophosphoric acid, gallium salt of vanadomolybdophosphoric acid, lithium salt of vanadomolybdosilicic acid, sodium salt of vanadomolybdosilicic acid, copper salt of vanadomolybdosilicic acid, gold salt of vanadomolybdosilicic acid and gallium salt of vanadomolybdosilicic acid.

Among these, preferred are lithium salt of tungstosilicic acid, sodium salt of tungstosilicic acid, copper salt of tungstosilicic acid, gold salt of tungstosilicic acid, gallium salt of tungstosilicic acid, lithium salt of tungstophosphoric acid, sodium salt of tungstophosphoric acid, copper salt of tungstophosphoric acid, gold salt of tungstophosphoric acid, gallium salt of tungstophosphoric acid, lithium salt of molybdophosphoric acid, sodium salt of molybdophosphoric acid, copper salt of molybdophosphoric acid, gold salt of molybdophosphoric acid, gallium salt of molybdophosphoric acid, lithium salt of molybdosilicic acid, sodium salt of molybdosilicic acid, copper salt of molybdosilicic acid, gold salt of molybdosilicic acid, gallium salt of molybdosilicic acid, lithium salt of vanadotungstosilicic acid, sodium salt of vanadotungstosilicic acid, copper salt of vanadotungstosilicic acid, gold salt of vanadotungstosilicic acid, gallium salt of vanadotungstosilicic acid, lithium salt of vanadotungstophosphoric acid, sodium salt of vanadotungstophosphoric acid, copper salt of vanadotungstophosphoric acid, gold salt of vanadotungstophosphoric acid and gallium salt of vanadotungstophosphoric acid.

More preferred are lithium salt of tungstosilicic acid, sodium salt of tungstosilicic acid, copper salt of tungstosilicic acid, gold salt of tungstosilicic acid, gallium salt of tungstosilicic acid, lithium salt of tungstophosphoric acid, sodium salt of tungstophosphoric acid, copper salt of tungstophosphoric acid, gold salt of tungstophosphoric acid, gallium salt of tungstophosphoric acid, lithium salt of vanadotungstosilicic acid, sodium salt of vanadotungstosilicic acid, copper salt of vanadotungstosilicic acid, gold salt of vanadotungstosilicic acid, gallium salt of vanadotungstosilicic acid, lithium salt of vanadotungstophosphoric acid, sodium salt of vanadotungstophosphoric acid, copper salt of vanadotungstophosphoric acid, gold salt of vanadotungstophosphoric acid and gallium salt of vanadotungstophosphoric acid.

In the catalyst for use in producing a lower aliphatic carboxylic acid ester of the present invention (I), the heteropolyacid salt as a catalytic activity component is supported on a support. Substances which can be used as the support are not particularly limited and porous substances commonly used as a support may be used. Specific examples thereof include those comprising silica, diatomaceous earth, montmorillonite, titania, activated carbon, alumina and silica alumina, preferably silica, silica alumina and montmorillonite.

The support is also not limited on the shape thereof and may be in the powder, spherical, pellet-like or any other form. A sphere or pellet-like form is preferred. Furthermore, the particle size is not particularly limited and although the preferred particle size varies depending on the reaction form, the average diameter is preferably from 2 to 10 mm in the case of use in a fixed bed system and from powder to 5 mm in the case of use in a fluidized bed system.

The support is most preferably a spherical or pellet-form siliceous support.

The method for loading the heteropolyacid salt on the support in the first step of the catalyst for producing a lower aliphatic carboxylic acid ester of the present invention (I) is roughly classified into the following three groups (1) to (3).
(1) A method of loading a desired heteropolyacid on a support and thereafter loading a starting material for the desired element or moiety of forming the salt.
(2) A method of loading a desired heteropolyacid together with a starting material for the element or moiety of forming the salt or loading a previously prepared heteropolyacid salt on a support.
(3) A method of previously loading a starting material for the element or moiety of forming the salt on a support and thereafter loading a desired heteropolyacid thereon.

In any of these methods (1) to (3), the heteropolyacid and the starting material for the element or moiety of forming the salt each can be loaded by dissolving or suspending it in an appropriate solvent. The solvent may be any as long as it can uniformly dissolve or suspend the desired heteropolyacid, a salt thereof and the starting material for the element or moiety of forming the salt, and examples of the solvent which can be used include water, an organic solvent and a mixture thereof. Among these, preferred are water, alcohols and carboxylic acids.

The method used for the dissolution or suspension may also be any as long as it can uniformly dissolve or suspend the desired heteropolyacid, a salt thereof and the starting material for the element or moiety of forming the salt. In the case of a free acid, a free acid which can dissolve may be dissolved as it is in a solvent and even in the case of a free acid which cannot completely dissolve, if the free acid can be uniformly suspended by forming it into fine powder, the free acid may be suspended as such.

In the method (1), a solution or suspension obtained by dissolving or suspending a heteropolyacid in a solvent is absorbed to a support to thereby load the heteropolyacid on the support and, then, a solution or suspension of a starting material for the element, or moiety of forming a desired salt is absorbed to the support to thereby load the element or moiety. At this time, a neutralization reaction proceeds on the support, as a result, a heteropolyacid salt supported catalyst can be prepared.

In the method (2), a heteropolyacid and a starting material for the element or moiety of forming the salt are dissolved or suspended together or separately and then mixed to prepare a uniform solution or suspension, and the solution or suspension is absorbed to a support, thereby loading the heteropolyacid and the element or moiety. In the case of a compound in the state of a heteropolyacid salt, a uniform solution or suspension may be obtained in the same manner as in the case of a free acid.

In the method (3), a solution or suspension of a starting material for the element or moiety of forming the salt is previously prepared, the solution or suspension is absorbed to a support to thereby load the element or moiety, and then a desired heteropolyacid is loaded. This method includes a method of using an element or moiety which is previously contained in the support and which can form a heteropolyacid salt.

More specifically, a part or all of the elements previously contained in a support sometimes act to form a salt of a heteropolyacid when the heteropolyacid is loaded, and as a result, a heteropolyacid salt is formed. Examples of such an element include potassium, sodium, calcium, iron, magnesium, titanium and aluminum, however, the present invention is not limited thereto.

The kind of the element previously contained in a support and the amount thereof can be determined by a chemical analysis such as inductively coupled plasma emission spectrometry (hereinafter referred to as "ICP"), fluorescent X-ray method and atomic absorption method. The kind and the amount of the element vary depending on the support, however, potassium, sodium, calcium, iron, magnesium, titanium and aluminum are sometimes contained in a relatively large amount and the content thereof is approximately from 0.001 to 5.0% by mass. Therefore, depending on the combination of a support and a heteropolyacid, the element previously contained in the support may be in an amount large enough to form a salt, though this may vary depending on the kind and the amount of the heteropolyacid to be supported.

The method for loading a solution or suspension of heteropolyacid or a salt thereof on a support is not particularly limited and a known method may be used. Specifically, for example, the catalyst may be prepared by dissolving a heteropolyacid in a distilled water corresponding to the liquid absorption amount of the support used and impregnating the solution into a support. Also, the catalyst may be prepared using an excess aqueous heteropolyacid solution by impregnating it into a support while appropriately moving the support in the heteropolyacid solution and then removing the excess acid by filtration. The volume of the solution or suspension used at this time varies depending on the support used or the loading method thereon.

The thus-obtained wet catalyst is suitably dried by placing it in a heating oven for a few hours. The drying method is not particularly limited and any method such as of a standing or belt conveyor system may be used. After drying, the catalyst is cooled to the ambient temperature in a desiccator so as not to absorb moisture.

The amount of the heteropolyacid salt supported in the heteropolyacid salt supported catalyst can be simply calculated by subtracting the weight of the support used from the weight after drying of the catalyst prepared. The amount supported may be more exactly determined by a chemical analysis such as ICP, fluorescent X-ray method or atomic absorption method.

The amount of the heteropolyacid salt supported is preferably from 10 to 150% by mass, more preferably from 30 to 100% by mass, based on the entire weight of the support.

If the heteropolyacid salt content is less than 10% by mass, the content of active components in the catalyst is too small and the activity per the catalyst unit weight may disadvantageously decrease. If the heteropolyacid salt content exceeds 150% by mass, the effective pore volume may decrease, as a result, the effect owing to the increase in the supported amount may not be brought out and at the same time, coking may be disadvantageously liable to occur to greatly shorten the catalyst life.

The second step is described below.

The second step in the catalyst for use in producing a lower aliphatic carboxylic acid ester of the present invention (I) is a step for contacting the heteropolyacid salt supported catalyst obtained in the first step with a gas containing at least one member selected from the group consisting of water, lower aliphatic carboxylic acids and lower aliphatic alcohols without any olefin.

The term "contacting" as used above refers to bringing the heteropolyacid salt supported catalyst obtained in the first step into contact with a gas containing at least one member selected from the group consisting of water, lower aliphatic carboxylic acids and lower aliphatic alcohols without any olefin. The method for the contacting is not particularly limited and for example, the following methods may be used.
(a) A method of placing the heteropolyacid salt supported catalyst obtained in the first step in an atmosphere of a gas containing at least one member selected from the group consisting of water, lower aliphatic carboxylic acids and lower aliphatic alcohols without any olefin.
(b) A method of passing a gas containing at least one member selected from the group consisting of water, lower aliphatic carboxylic acids and lower aliphatic alcohols without any olefin through the heteropolyacid salt supported catalyst obtained in the first step.
(c) A method of passing the heteropolyacid salt supported catalyst obtained in the first step, through an atmosphere of a gas containing at least one member selected from the group consisting of water, lower aliphatic carboxylic acids and lower aliphatic alcohols without any olefin.

These methods may be used in combination of two or more thereof.

To speak more specifically on the method of performing the second step, for example, a method of filling the supported catalyst obtained in the first step into a vessel and contacting the gas therewith, or a method of filling the supported catalyst obtained in the first step into, in place of the vessel, a reactor where the production process of a lower aliphatic carboxylic acid ester is performed later, and contacting the gas therewith before feeding reaction starting materials, may be used.

with respect to the shape of the vessel or the reactor used here, any shape such as vertical type or horizontal type may be used without any particular limit.

In view of the time to be spent for re-filling the catalyst or the vessel cost, the preferred embodiment of the second step includes a method of filling the supported catalyst obtained in the first step into a reactor which is used in reacting a lower olefin with a lower aliphatic carboxylic acid in a gas phase to produce a lower aliphatic carboxylic acid ester, and then contacting therewith a gas containing at least one member selected from the group consisting of water, lower aliphatic carboxylic acids and lower aliphatic alcohols without any olefin before feeding the reaction starting materials. At this time, the reaction may be performed in either a closed circulatory system or a flow system.

The second step is preferably performed under conditions not less than the dew point of the gas containing at least one member selected from the group consisting of water, lower aliphatic carboxylic acids and lower aliphatic alcohols without any olefin. If the conditions are less than the dew point of the gas, a part of the gas may turn into a liquid. In this case, the heteropolyacid salt supported on the catalyst in the first step or other catalyst components supported, as desired, may dissolve out to change the catalyst composition, and in the worst case, the catalyst may be deactivated. As long as the catalyst is not adversely affected, the conditions in performing the second step are not particularly limited.

The preferred embodiment of the conditions at the dew point or more of the gas may vary depending on the composition of the gas or the pressure or the like in the practice of the step, however, the contact temperature is preferably from 80 to 300°C, more preferably from 100 to 260°C.

The contact pressure is not particularly limited and may be either normal pressure or raised pressure. The contact pressure is preferably from 0 to 3 MPaG (gauge pressure), more preferably from 0 to 2 MPaG (gauge pressure).

The lower aliphatic carboxylic acid in a gas containing at least one member selected from the group consisting of water, lower aliphatic carboxylic acids and lower aliphatic alcohols without any olefin used in the second step, is a lower aliphatic carboxylic acid having from 1 to 6 carbon atoms. Specific examples thereof include formic acid, acetic acid, propionic acid, n-butyric acid and isobutyric acid. Among these, preferred are acetic acid and propionic acid.

The lower aliphatic alcohol in a gas containing at least one member selected from the group consisting of water, lower aliphatic carboxylic acids and lower aliphatic alcohols without any olefin used in the second step is a lower aliphatic alcohol having from 1 to 6 carbon atoms. Specific examples thereof include methanol, ethanol, 1-propanol, 2-propanol, 1-butanol and 2-butanol. Among these, preferred are methanol, ethanol and n-propanol.

The composition of the gas containing at least one member selected from the group consisting of water, lower aliphatic carboxylic acids and lower aliphatic alcohols without any olefin used in the second step is not particularly limited, and water, a lower aliphatic carboxylic acid and a lower aliphatic alcohol can be mixed at any ratio. The composition is preferably such that water : a lower aliphatic carboxylic acid : a lower aliphatic alcohol = 1.0 : 0.1-10.0 : 0.1-5.0 by molar ratio.

The composition of the gas may be constant from the beginning to the end of contacting or may vary according to the contacting time or the stage of contacting.

The gas containing at least one member selected from the group consisting of water, lower aliphatic carboxylic acids and lower aliphatic alcohols without any olefin used in the second step is more preferably water alone or a mixed gas of water and acetic acid, still more preferably a mixed gas of water and acetic acid because the effect can be provided within a short time.

In the case of using a mixed gas of water and acetic acid as the gas, the composition is not particularly limited but preferably such that water : acetic acid = 1.0 : 0.1-10.0, more preferably water : acetic acid = 1.0 : 0.5-5.0, by molar ratio.

The gas hourly space velocity (hereinafter referred to as "GHSV"), which is the speed of feeding the gas in performing the contact with a gas containing at least one member selected from the group consisting of water, lower aliphatic carboxylic acids and lower aliphatic alcohols used in the second step, is not particularly limited. The GHSV is preferably from 100 to 7,000 hr⁻¹, more preferably from 300 to 3,000 hr⁻¹.

If the GHSV is too high, the amount of the gas used may increase and this is not preferred in view of the cost. From this standpoint, the contacting may also be performed by feeding a constant amount of the gas and enclosing it in a vessel.

The contacting time is not particularly limited, however, it is preferably from 0.5 to 200 hours, more preferably from 0.5 to 100 hours, and most preferably from 0.5 to 50 hours. The optimal time varies depending on the composition and concentration of the gas, the temperature and pressure at the contacting, and the catalyst component.

Generally, if the contacting time is less than 0.5 hours, the effect of the second step may not be fully brought out, whereas if the contacting time is prolonged, the effect may be liable to increase but even if the contacting time is prolonged to exceed 200 hours, the effect may not increase any more, moreover, in the case where gas is contacted in the flowing state, the amount of the gas used may increase and this is not preferred in view of the profitability.

The present invention (II) is described below. The present invention (II) is a process for producing a catalyst for use in producing a lower aliphatic carboxylic acid ester, the catalyst being used in reacting a lower olefin with a lower aliphatic carboxylic acid in a gas phase to produce a lower aliphatic carboxylic acid ester, which process comprises the following first and second steps:

### First Step

a step for loading one or more heteropolyacid salts on a support to obtain a heteropolyacid salt supported catalyst; and

### Second Step

a step for contacting the heteropolyacid salt supported catalyst obtained at the first step with a gas containing at least one member selected from the group consisting of water, lower aliphatic carboxylic acids and lower aliphatic alcohols without any olefin to obtain a catalyst for use in producing a lower aliphatic carboxylic acid ester.

The heteropolyacid salt and the support used in the first step, the method for loading the heteropolyacid salt on a support performed in the first step, the method for measuring the amount of the heteropolyacid salt supported, and the preferred amount of the heteropolyacid salt supported on a support may be the same as in the present invention (I).

Furthermore, the method for contacting the catalyst with a gas containing at least one member selected from the group consisting of water, lower aliphatic carboxylic acids and lower aliphatic alcohols without any olefin performed in the second step, the conditions therefor such as temperature, pressure, GHSV and time, the lower aliphatic carboxylic acid and lower aliphatic alcohol used for the gas, and the compositional ratio of the gas to which water is further added, may be the same as in the present invention (I).

In the process for producing a catalyst for use in producing a lower aliphatic carboxylic acid ester of the present invention (II), as long as the following first and second steps are contained, other steps may be provided before, after or during these steps, if desired.

### First Step

A step for loading one or more heteropolyacid salts on a support to obtain a heteropolyacid salt supported catalyst.

### Second Step

A step for contacting the heteropolyacid salt supported catalyst obtained in the first step with a gas containing at least one member selected from the group consisting of water, lower aliphatic carboxylic acids and lower aliphatic alcohols without any olefin to obtain a catalyst for use in producing a lower aliphatic carboxylic acid ester.

Examples of the step which is provided, as desired, include a step for loading a third component so as to more improve the catalytic activity. In such a case, this loading operation may be performed simultaneously with the operation of loading the heteropolyacid salt in the first step, if possible.

After the second step of contacting with a gas containing at least one member selected from the group consisting of water, lower aliphatic carboxylic acids and lower aliphatic alcohols without any olefin, the catalyst may further be contacted with another gas.

The present invention (III) is described below. The present invention (III) is a process for producing a lower aliphatic carboxylic acid ester, comprising reacting a lower olefin with a lower aliphatic carboxylic acid in a gas phase in the presence of the catalyst for use in producing a lower aliphatic carboxylic acid ester of the present invention (I).

In the process for producing a lower aliphatic carboxylic acid ester of the present invention (III), a gas phase reaction is not particularly limited on the reaction form and the reaction may be performed in any form such as in a fixed bed system or fluidized bed system. The shape of the support which governs the shape or size of the catalyst may be selected in the range from powder to a compact formed into a size of several mm according to the reaction form employed.

The lower olefin which is used in the process for producing a lower aliphatic carboxylic acid ester of the present invention (III) are ethylene, propylene, n-butene, isobutene and a mixture of two or more thereof.

The lower aliphatic carboxylic acid is a carboxylic acid having from 1 to 4 carbon atoms and specific examples thereof include formic acid, acetic acid, propionic acid, butyric acid, acrylic acid and methacrylic acid.

The ratio between the lower olefin and the lower aliphatic carboxylic acid used as the starting materials is not particularly limited. In view of the conversion of the lower olefin, the lower olefin is preferably used in an equimolar or excess amount based on the lower aliphatic carboxylic acid. Specifically, the molar ratio of the lower olefin to the lower aliphatic carboxylic acid is preferably in the range of lower olefin : a lower aliphatic carboxylic acid = from 1:1 to 30:1, more preferably from 3:1 to 20:1, still more preferably from 5:1 to 15:1.

Furthermore, from the standpoint of maintaining the catalytic activity, a small amount of water is preferably added to the starting materials. However, if an excessively large amount of water is added, the amount of a by-product such as an alcohol or an ether may disadvantageously increase. The amount of water added is preferably from 0.5 to 15 mol%, more preferably from 2 to 8 mol%, in terms of the molar ratio of water to the total amount of the lower olefin, the lower aliphatic carboxylic acid, as the starting materials, and water added.

The reaction conditions such as temperature and pressure preferred in the process for producing a lower aliphatic carboxylic acid ester of the present invention (III), may vary depending on the lower olefin and the lower aliphatic carboxylic acid used as the starting materials. The reaction conditions such as temperature and pressure are preferably combined so that the starting materials each can be kept in the gas state and the reaction can satisfactorily proceed.

In general, the temperature is preferably from 120 to 300°C, more preferably from 140 to 250°C. The pressure is preferably from 0 to 3 MPaG (gauge pressure), more preferably from 0 to 2 MPaG (gauge pressure).

The starting materials each is not particularly limited on the GHSV. If the GHSV is excessively high, the gas may pass through before the reaction satisfactorily proceeds, whereas if it is too low, problems, such as reduction in the productivity, may arise. The GHSV is preferably from 100 hr⁻¹ to 7,000 hr⁻¹ , more preferably from 300 hr⁻¹ to 3,000 hr⁻³.

The unreacted lower olefin and also alcohol and ether produced as by-products in the reaction may be recycled and used as they are. At this time, the substances which are harmful to the catalyst for use in producing a lower aliphatic carboxylic acid ester, such as butene and an aldehyde, are difficult to separate from olefin, alcohol, ether and the like, and may sometimes be transferred to a reactor. In such a case, the catalyst may be conspicuously reduced in activity or extremely shortened in the life. The process for producing a lower aliphatic carboxylic acid ester of the present invention (III) using the catalyst for use in producing a lower aliphatic carboxylic acid ester of the present invention (I) can greatly decrease the production of those by-products at the stage of reaction, therefore, the present invention (III) is particularly effective in the case where the above-described recycling system is employed in the production process.

The present invention (IV) is described below. The present invention (IV) is a process for producing a lower aliphatic carboxylic acid ester, comprising reacting a lower olefin with a lower aliphatic carboxylic acid in a gas phase, which process comprises the following first to fourth steps:

### First Step

a step of loading one or more heteropolyacid salts on a support to obtain a heteropolyacid salt supported catalyst;

### Second Step

a step of filling the heteropolyacid salt supported catalyst obtained in the first step, into a reactor for use in the reaction of a lower olefin with a lower aliphatic carboxylic acid in a gas phase;

### Third Step

a step of contacting the heteropolyacid salt supported catalyst filled in the reactor, with a gas containing at least one member selected from the group consisting of water, lower aliphatic carboxylic acids and lower aliphatic alcohols without any olefin; and, distinguished from the Third Step, as a

### Fourth Step

a step of passing a mixed gas containing the lower olefin and the lower aliphatic carboxylic acid through the heteropolyacid salt supported catalyst after the third step, to obtain the lower aliphatic carboxylic acid ester.

The first step is described below. The first step in the process for producing a lower aliphatic carboxylic acid ester of the present invention (IV) is a step of loading a heteropolyacid salt on a support to obtain a heteropolyacid salt supported catalyst.

The heteropolyacid salt and the support used in the first step, the method for loading the heteropolyacid salt on a support performed in the first step, the method for measuring the amount of the heteropolyacid salt supported, and the preferred amount of the heteropolyacid salt supported on a support may be the same as in the present invention (I).

The second step is described below. The second step in the process for producing a lower aliphatic carboxylic acid ester of the present invention (IV) is a step of filling the heteropolyacid salt supported catalyst obtained in the first step, into a reactor for use in reacting a lower olefin with a lower aliphatic carboxylic acid in a gas phase.

The second step is a step of filling the heteropolyacid salt supported catalyst into a reactor for use in the reaction of a lower olefin with a lower aliphatic carboxylic acid, so that the second step of the present invention (I) which is the catalyst for use in producing a lower aliphatic carboxylic acid ester, or the second step of the present invention (II) which is the process for producing a catalyst for use in producing a lower aliphatic carboxylic acid ester, namely, a step of contacting the heteropolyacid salt supported catalyst obtained in the first step with a gas containing at least one member selected from the group consisting of water, lower aliphatic carboxylic acids and lower aliphatic alcohols without any olefin, can be performed in the reactor without using another vessel.

The reactor used in the second step is not particularly limited. A fixed bed gas phase-type reactor is preferred, and a reactor having a form of multi-tube system and/or multi-layer system is more preferred. In general, a reactor having a form of multi-tube system and/or multi-layer system is superior in the reaction results, thermal efficiency, eases of control and the like. Of course, the present invention is not limited thereto.

In the present invention, the term "filling the catalyst into a reactor" refers to placing the catalyst in a predetermined site of the reactor. The site and the method for the placement and in the case where the reactor uses a fixed bed system, the method for fixing the catalyst may vary depending on the form of the reactor and these are not particularly limited. Specific examples of the reactor include Fig. C·4·43 "Methanol Treating Gas Phase Reactor" in the item of "4) Fixed catalyst gas phase reactor", described in Kagaku Sochi Binran (Chemical Apparatuses Handbook), 2nd ed., 3rd imp., pp. 905 to 906, edited by Society of Japan Chemical Engineering, published by Maruzen (February 20, 1980).

The third step is described below. The third step in the process for producing a lower aliphatic carboxylic acid ester of the present invention (IV) is a step of contacting a gas containing at least one member selected from the group consisting of water, lower aliphatic carboxylic acids and lower aliphatic alcohols without any olefin with the heteropolyacid salt supported catalyst filled in the reactor.

The method for contacting the catalyst with a gas containing at least one member selected from the group consisting of water, lower aliphatic carboxylic acids and lower aliphatic alcohols without any olefin performed in the third step, the conditions such as temperature, pressure, space velocity and time, the lower aliphatic carboxylic acid and lower aliphatic alcohol used for the gas, and the compositional ratio of the gas to which water is further added, may be the same as the second step of the present invention (I).

In performing the contacting in the reactor, the conditions therefor are not particularly limited, and the preferred conditions are considered to vary depending on the form, shape, size or constructive material of the reactor used for the contacting. In general, the contacting may be performed under the conditions described above for the second step of the present invention (I).

The fourth step is described below. The fourth step of the present invention (IV) is a step of passing a mixed gas containing the lower olefin and the lower aliphatic carboxylic acid through the heteropolyacid salt supported catalyst after the third step, to obtain the lower aliphatic carboxylic acid ester.

The lower olefin and lower aliphatic carboxylic acid used in the fourth step, the amount ratio thereof, the conditions such as addition of water, temperature, pressure and GHSV on performing the fourth step, and the recycling operation mainly of the unreacted lower olefin may be the same as in the process for the producing a lower aliphatic carboxylic acid ester of the present invention (III).

The third step and the fourth step of the present invention (IV) are clearly distinguished. After judging that the contacting in the third step is finished, the flow of the gas containing at least one member selected from the group consisting of water, lower aliphatic carboxylic acids and lower aliphatic alcohols without any olefin is once stopped or the temperature is further lowered to complete the third step and, thereafter, the starting material gas containing a lower olefin and a lower aliphatic carboxylic acid is passed as the reaction gas in the fourth step.

In general, the contacting in the third step is preferably performed using a lower aliphatic carboxylic acid corresponding to the ester as an objective product of the process for producing a lower aliphatic carboxylic acid ester of the present invention (IV). For example, in the case of producing ethyl acetate as the lower aliphatic carboxylic acid ester by applying the process for producing a lower fatty acid ester of the present invention (IV), one of the preferred practical embodiments is a method of performing the contacting in the third step using a mixed gas of water and acetic acid for a constant time under constant conditions, then varying various conditions within the reactor, such as temperature, pressure and GHSV, to fit the production process of a lower aliphatic carboxylic acid ester, and adding ethylene to the gas introduced into the reactor. Of course, the present invention (IV) is not limited thereto.

The present invention will be further illustrated below by referring to the Examples and Comparative Examples, however, the present invention should not be construed as being limited thereto. Conditions for Analysis of Metal in Support

The analysis of metals in the support was performed using a fluorescent X-ray analyzer (PW2404, manufactured by PHILIPS). Measurement conditions - atmosphere: helium, effective diameter: 25.0 mm, and matrix: 2 SiO₂. Conditions for Analysis of Uncondensed Gas

The analysis was performed under the following conditions using an absolute calibration curve process by sampling 50 ml of the effluent gas and passing the entire amount thereof into a 1 ml gas sampler attached to the gas chromatograph.
1. Ether, Carboxylic Acid Ester, Alcohol and Trace ByProducts
   - Gas chromatography:: gas chromatography (GC-14B, manufactured by Shimadzu Seisakusho Co.) with a gas sampler for Shimadzu gas chromatograph (MGS-4, measuring tube: 1 ml)
   - Column:: packed column SPAN 80, 15% Shinchrom A of 60 to 80 mesh (length: 5 m)
   - Carrier gas:: nitrogen (flow rate: 25 ml/min)
   - Temperature conditions:: The detector and the vaporization chamber were at a temperature of 120°C, and the column temperature was 65°C and constant.
   - Detector:: FID (H₂ pressure: 60 kPa, air pressure: 100 kPa)

### 2. Butene

- Gas chromatography:: gas chromatography (GC-14B, manufactured by Shimadzu Seisakusho Co.) with a gas sampler for Shimadzu gas chromatograph (MGS-4, measuring tube: 1 ml)
- Column:: packed column Unicarbon A-400 of 80/100 mesh, length: 2 m
- Carrier gas:: helium (flow rate: 23 ml/min)
- Temperature condition:: The detector and the vaporization chamber were at a temperature of 130°C, and the column temperature was elevated from 40°C to 95°C at a temperature rising rate of 40°C/min.
- Detector:: FID (H₂ pressure: 70 kPa, air pressure: 100 kPa)

### 3. Ethylene

- Gas chromatography:: gas .chromatography (GC-14B, manufactured by Shimadzu Seisakusho Co.) with a gas sampler for Shimadzu gas chromatograph (MGS-4, measuring tube: 1 ml)
- Column:: packed column Unibeads IS, length: 3 m
- Carrier gas:: helium (flow rate: 20 ml/min)
- Temperature condition:: The detector and the vaporization chamber were at a temperature of 120°C, and the column temperature was 65°C and constant.
- Detector:: TCD (He pressure: 70 kPa, current: 90 mA, temperature: 120°C)

### Analysis of Solution Collected

The analysis was performed using an internal standard method by injecting 0.4 µl of an analysis solution obtained by adding 1 ml of 1,4-dioxane as an internal standard to 10 ml of the reaction solution.
- Gas chromatography:: GC-14B manufactured by Shimadzu Seisakusho Co.
- Column:: capillary column TC-WAX (length: 30 m, inner diameter: 0.25 mm, film thickness: 0.25 µm)
- Carrier gas:: nitrogen (split ratio: 20, column flow rate: 1 ml/min)
- Temperature condition:: The detector and the vaporization chamber were at a temperature of 200°C, and the column temperature was kept at 50°C for 5 minutes from the starting of analysis, then elevated to 150°C at a temperature rising rate of 20°C/min, and kept at 150°C for 10 minutes.
- Detector:: FID (H₂ pressure: 70 kPa, air pressure: 100 kPa)

### Support

- Support 1:: synthetic silica (N-602T, produced by Nikki Kagaku K.K.) (specific surface area: 132 m²/g, pore volume: 0.7 cm³/g)
- Support 2:: synthetic silica (CARiACT Q-10, produced by Fuji Silicia Kagaku K.K.) (specific surface area: 219.8 m²/g, pore volume: 0.660 cm³/g)
- Support 3:: natural silica (KA-160, produced by Sud Chemie AG) (specific surface area: 130 m²/g, pore volume: 0.53 cm³/g)
- Support 4:: natural silica (KA-0, produced by Sud Chemie AG) (specific surface area: 68.5 m²/g, pore volume: 0.71 cm³/g)
- Support 5:: silica gel (Wakogel C-200, produced by Wako Junyaku Kogyo K.K.) (specific surface area: 762 m²/g, pore volume: 0.23 cm³/g)

The metal analysis results of each support are shown in Table 1.

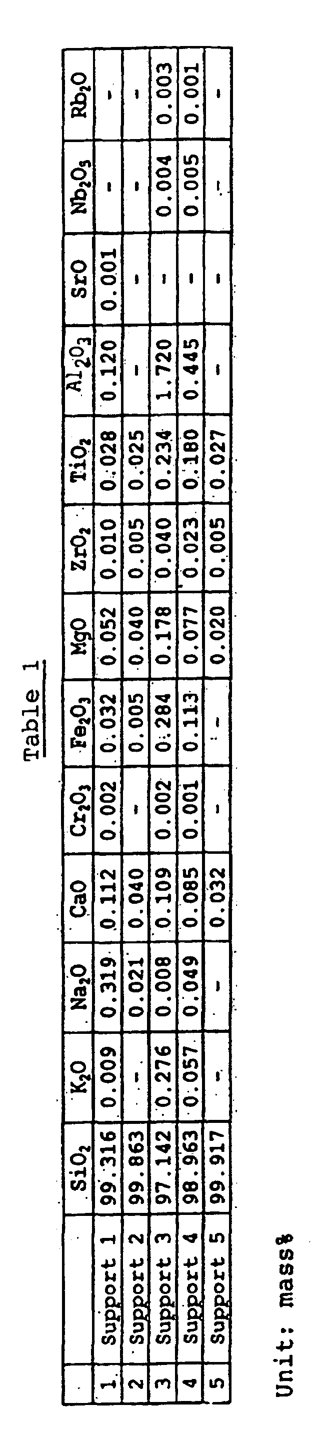

### Preparation Process of Catalyst 1

Support 1 was preliminarily dried for 4 hours in a (hot-air type) drier previously adjusted to 110°C. After the preliminary drying, 1 liter of the support was measured on the bulk density using a 1 liter measuring cylinder. Tungstosilicic acid was weighed to the amount shown in Table 2 and after adding thereto 15 ml of pure water, uniformly dissolved. To this impregnating solution, pure water was further added to make the liquid amount shown in Volume of Solution Prepared of Table 2. Thereafter, the preliminarily dried support was weighed to the amount shown in Table 2, added to the impregnating solution and impregnated with the solution while thoroughly stirring. The support impregnated with the solution was transferred to a porcelain dish, air-dried for 1 hour and then dried in a hot-air type drier adjusted to 150 °C for 5 hours. After the drying, the catalyst was transferred in a desiccator and left standing to cool to room temperature. This is designated as a "tungstosilicic acid supported catalyst".

Subsequently, lithium nitrite was weighed to the amount shown in Table 2 and after adding thereto 15 ml of pure water, uniformly dissolved. To this impregnating solution, pure water was added to make the liquid amount shown in Volume of Solution Prepared shown of Table 2 and then the solution was uniformly stirred. The tungstosilicic acid supported catalyst after cooling was added, in the entire amount, to the impregnating solution and impregnated with the solution while thoroughly stirring. The support impregnated with the solution was transferred to a porcelain dish, air-dried for 1 hour and then dried in a hot-air type drier adjusted to 150°C for 5 hours. After the drying, the catalyst was transferred in a desiccator and left standing to cool to room temperature. The weight of the thus-obtained catalyst was measured.

The result is also shown in Table 2.

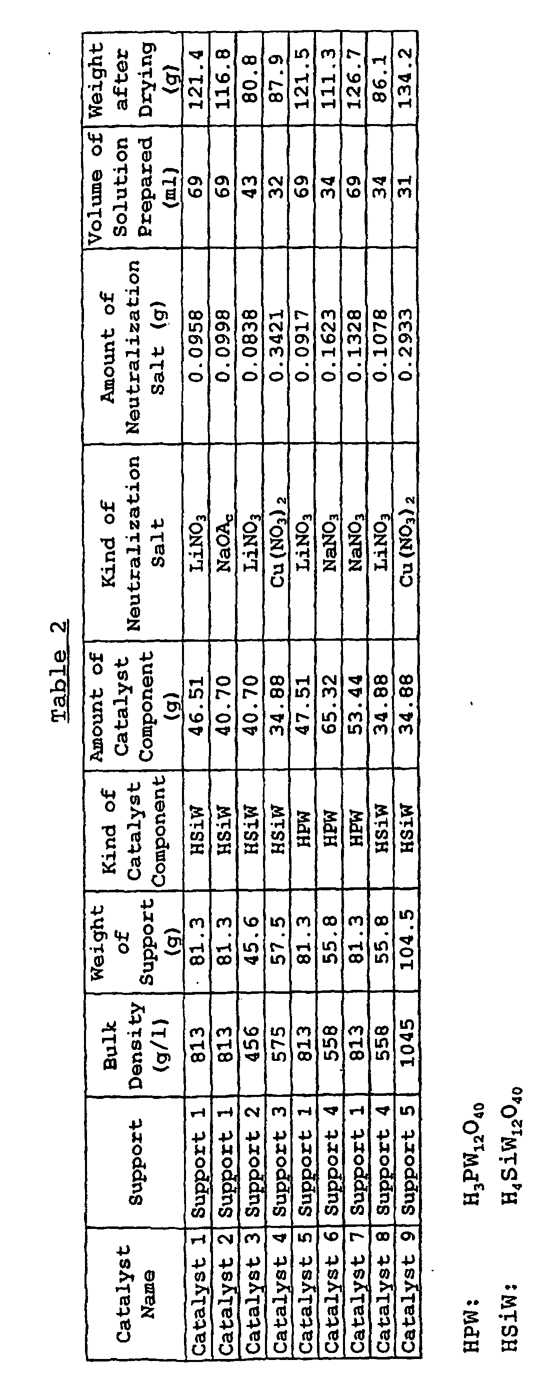

### Preparation Process of Catalyst 2

Support 1 was preliminarily dried for 4 hours in a (hot-air type) drier previously adjusted to 110°C. After the preliminary drying, 1 liter of the support was measured on the bulk density using a 1 liter measuring cylinder. Tungstosilicic acid and sodium acetate each was weighed to the amount shown in Table 2 and after adding thereto 15 ml of pure water, uniformly dissolved to obtain an aqueous solution of Na_{0.1}H_{3.9}SiW₁₂O₄₀ (impregnating solution). To this impregnating solution, pure water was further added to make the liquid amount shown in Volume of Solution Prepared of Table 2. Thereafter, the preliminarily dried support was weighed to the amount shown in Table 2, added to the impregnating solution and impregnated with the solution while thoroughly stirring. The support impregnated with the solution was transferred to a porcelain dish, air-dried for 1 hour and then dried in a hot-air type drier adjusted to 150°C for 5 hours. After the drying, the catalyst was transferred in a desiccator and left standing to cool to room temperature. The weight of the thus-obtained catalyst was measured.

The result is also shown in Table 2.

### Preparation Process of Catalysts 3, 5 and 7

These catalysts were prepared by changing the kind and weight of the support, the kind and weight of the catalyst component, and the weight of the neutralization salt as shown in Table 2 in Preparation Process of Catalyst 1. The loading on a support was performed in the same manner as in Preparation Process of Catalyst 1.

The results are also shown in Table 2. Preparation Process of Catalysts 4 and 6

These catalysts were prepared by changing the kind and weight of the support, the kind and weight of the catalyst component, and the weight of the neutralization salt as shown in Table 2 in Preparation Process of Catalyst 2. The loading on a support was performed in the same manner as in Preparation Process of Catalyst 2.

The results are also shown in Table 2.

### Preparation Process of Catalyst 8

Support 4 was preliminarily dried for 4 hours in a (hot-air type) drier previously adjusted to 110°C. After the preliminary drying, 1 liter of the support was measured on the bulk density using a 1 liter measuring cylinder. Lithium nitrate was weighed to the amount shown in Table 2 and after adding thereto 15 ml of pure water, uniformly dissolved. To this impregnating solution, pure water was further added to make the liquid amount shown in Volume of Solution Prepared of Table 2. Thereafter, the preliminarily dried support was weighed to the amount shown in Table 2, added to the impregnating solution and impregnated with the solution while thoroughly stirring. The support impregnated with the solution was transferred to a porcelain dish, air-dried for 1 hour and then dried in a hot-air type drier adjusted to 150°C for 5 hours. After the drying, the catalyst was transferred in a desiccator and left standing to cool to room temperature. This is designated as a "lithium nitrate supported catalyst". Tungstosilicic acid was weighed to the amount shown in Table 2 and after adding thereto 15 ml of pure water, uniformly dissolved.

To this impregnating solution, pure water was added to make the liquid amount shown in Volume of Solution Prepared shown of Table 2 and then the solution was uniformly stirred. The lithium nitrate supported catalyst was added in the entire amount to the impregnating solution and impregnated with the solution while thoroughly stirring. The lithium nitrate supported catalyst impregnated with the solution was transferred to a porcelain dish, air-dried for 1 hour and then dried in a hot-air type drier adjusted to 150°C for 5 hours. After the drying, the catalyst was transferred in a desiccator and left standing to cool to room temperature. The weight of the thus-obtained catalyst was measured.

The result is also shown in Table 2.

### Preparation Process of Catalyst 9

This catalyst was prepared by changing the kind and weight of the support, the kind and weight of the catalyst component, and the weight of the neutralization salt as shown in Table 2 in Preparation Process of Catalyst 8. The loading on a support was performed in the same manner as in Preparation Process of Catalyst 8.

The result is also shown in Table 2.

### Example 1

Into a pressure resistant vessel made of SUS 316L, 50 ml of the catalyst obtained in Preparation Process of Catalyst 1 was filled, and then contacted under the contacting conditions shown in Table 3. After the contacting, the vessel was cooled to room temperature and the catalyst used for the contacting (hereinafter referred to as "contacted catalyst") was drawn out from the vessel. Subsequently, 40 ml of the contacted catalyst was filled into a reaction tube, and a mixed gas of ethylene : acetic acid : steam : nitrogen (=78.5:8.0:4.5:9.0 by volume) was introduced thereinto at a temperature of 165°C, a pressure of 0.8 MPaG (gauge pressure) and a GHSV of 1,500 hr⁻¹, and reacted. The gas passed through the catalyst layer was collected under ice cooling for a predetermined time and the entire amount of condensed components (hereinafter referred to as a "condensed solution") was recovered and analyzed. The outlet gas remaining uncondensed (hereinafter referred to as an "uncondensed gas") was measured on the gas flow rate for the same predetermined time as in the condensed solution and then 50 ml of the gas was sampled and analyzed. The results are shown in Table 3.

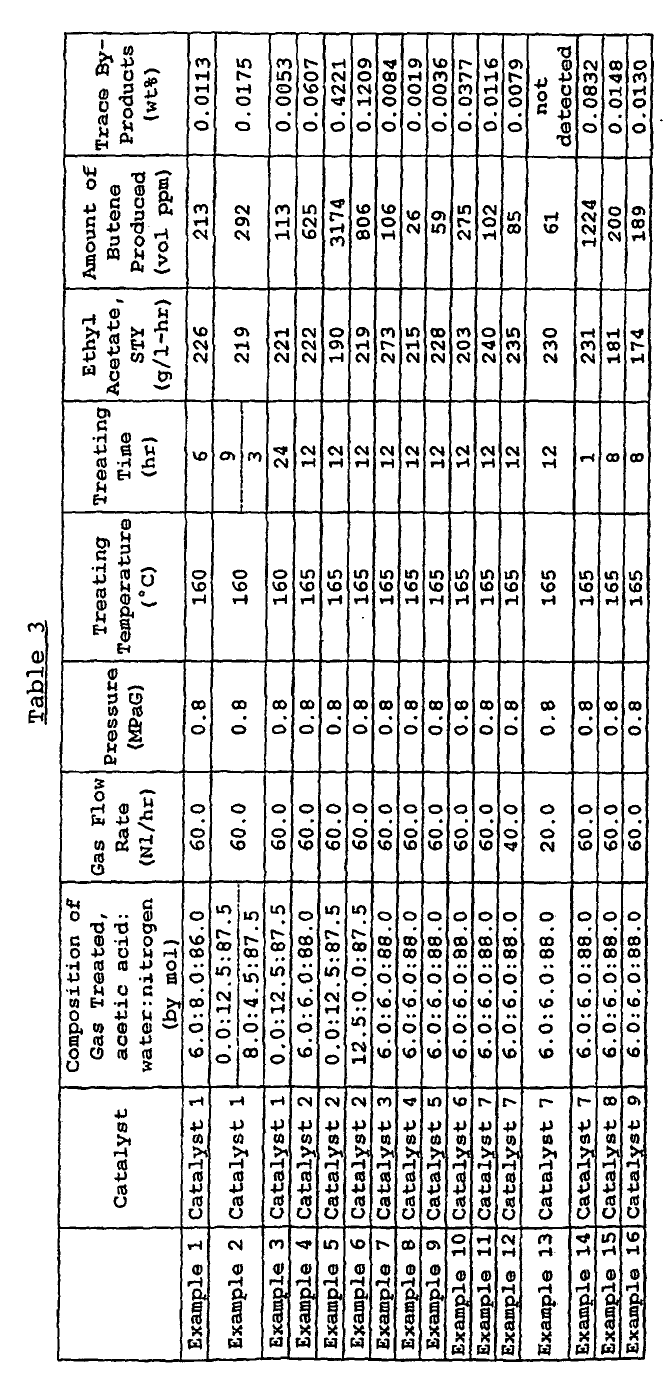

### Examples 2 and 3

In the same manner as in Example 1, 50 ml of the catalyst obtained in Preparation Process of Catalyst 1 was filled into a pressure resistant vessel made of SUS 316L and contacted under the contacting conditions shown in Table 3. Thereafter, the catalyst was drawn out, filled into a reaction tube and then subjected to a reaction in the same manner. The results are shown in Table 3.

### Example 4

Into a reaction tube, 40 ml of the catalyst obtained in Preparation Process of Catalyst 2 was filled and contacted under the contacting conditions shown in Table 3. Subsequently, a mixed gas of ethylene : acetic acid : steam : nitrogen (=78.5:8.0:4.5:9.0 by volume) was introduced thereinto at a temperature of 165°C, a pressure of 0.8 MPaG (gauge pressure) and a GHSV of 1,500 hr⁻¹, and reacted. The reaction results are shown in Table 3.

### Examples 5 to 16

In the same manner as in Example 4, 40 ml of the catalyst shown in Table 3 was filled into a reaction tube and contacted under the contacting conditions shown in Table 3. Thereafter, a reaction was performed in the same manner as in Example 5. The reaction results are shown in Table 3.

### Comparative Example 1

Into a reaction tube, 40 ml of the same catalyst as in Example 1 was filled and without performing contacting, a mixed gas of ethylene : acetic acid : steam : nitrogen (=78.5:8.0:4.5:9.0 by volume) was introduced thereinto at a temperature of 165°C, a pressure of 0.8 MPaG (gauge pressure) and a GHSV of 1,500 hr⁻¹, and reacted. The gas passed through the catalyst layer was collected and analyzed in the same manner as in Example 1. The results are shown in Table 4.

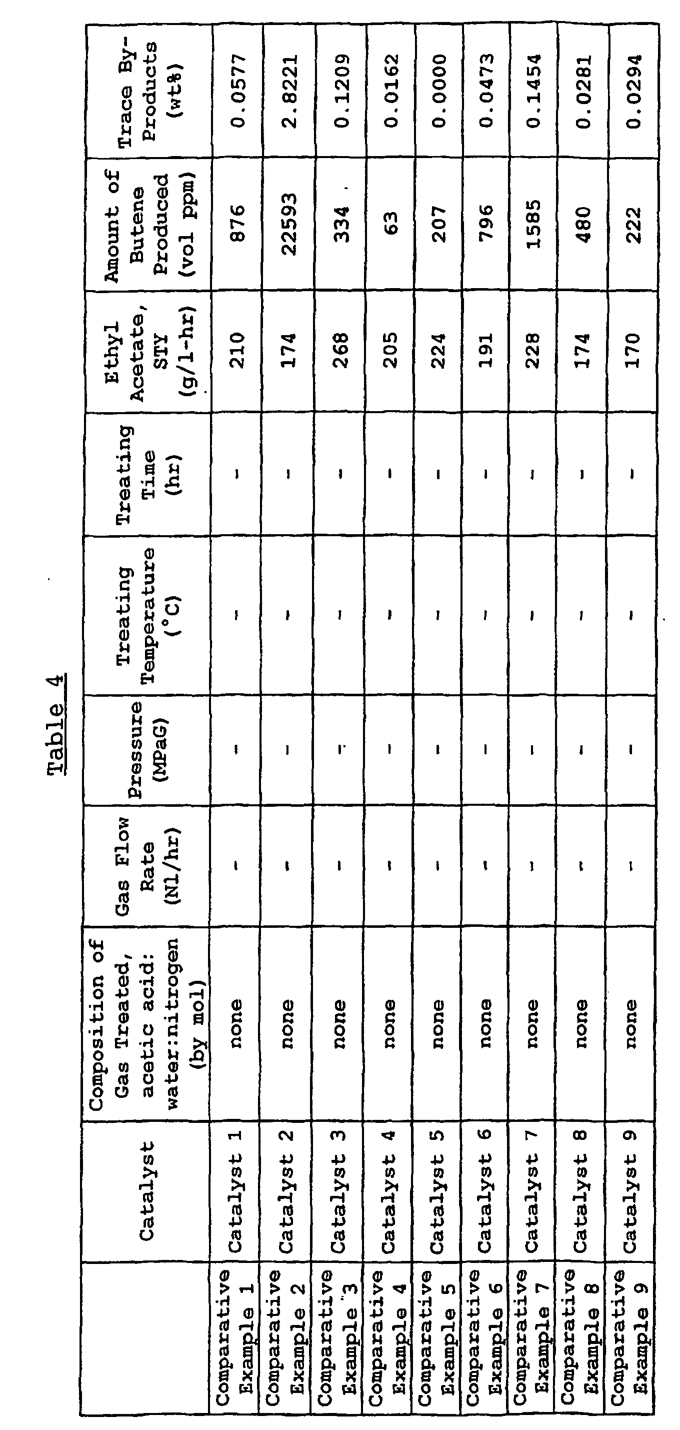

### Comparative Examples 2 to 9

In the same manner as in Comparative Example 1, 40 ml of the catalyst shown in Table 3 was filled into a reaction tube and a reaction was performed. The reaction results are shown in Table 4.

### Industrial Applicability

It is apparent from the above results that in producing a lower aliphatic carboxylic acid ester from a lower olefin and a lower aliphatic carboxylic acid using a heteropolyacid salt as a catalyst, when the catalyst is contacted with a gas containing at least one member selected from the group consisting of water, lower aliphatic carboxylic acids and lower aliphatic alcohols without any olefin before performing the reaction, the catalyst obtained can exhibit high initial activity and high space time yield, ensure a sufficiently long catalyst life in the practice in industry, and be greatly prevented from production of by-product compounds harmful to the catalyst, such as butene and aldehydes.

This catalyst can maintain its catalytic activity without performing the removal of by-products, therefore, the catalyst is very useful particularly in practicing the process for producing a lower aliphatic carboxylic acid ester by employing a recycling system.

## Claims

1. A catalyst for use in producing a lower aliphatic carboxylic acid ester, which is used in reacting an olefin selected from the group consisting of ethylene, propylene, n-butene, isobutene and a mixture of two or more thereof with an aliphatic carboxylic acid having 1 to 4 carbon atoms in the gas phase to produce a lower aliphatic carboxylic acid ester, wherein the catalyst is obtainable by a process comprising the following first and second steps:
First Step
a step for loading one or more heteropolyacid salts on a support to obtain a heteropolyacid salt supported catalyst; and
Second Step
a step for contacting the heteropolyacid salt supported catalyst obtained in the first step with a gas containing at least one member selected from the group consisting of water, aliphatic carboxylic acids having 1 to 6 carbon atoms and aliphatic alcohols having 1 to 6 carbon atoms without any olefin to obtain the catalyst for use in producing a lower aliphatic carboxylic acid ester.

2. The catalyst as claimed in claim 1, wherein the heteropolyacid salt is at least one salt selected from the group consisting of lithium salts, sodium salts, magnesium salts, barium salts, copper salts, gold salts and gallium, salts of silicotungstic acid, phosphotungstic acid, phosphomolybdic acid, silicomolybdic acid, silicovanadotungstic acid, phosphovanadotungstic acid and phosphovanadomolybdic acid.

3. A process f or producing a catalyst for use in producing a lower aliphatic carboxylic acid ester said catalyst being used in reacting an olefin selected from the group consisting of ethylene, propylene, n-butene, isobutene and a mixture of two or more thereof with an aliphatic carboxylic acid having 1 to 4 carbon atoms in the gas phase to produce a lower aliphatic carboxylic acid ester, which process comprises the following first and second steps:
First Step
a step for loading one or more heteropolyacid salts on a support to obtain a heteropolyacid salt supported catalyst; and
Second Step
a step for contacting the heteropolyacid salt supported catalyst obtained in the first step with a gas containing at least one member selected from the group consisting of water, aliphatic carboxylic acids having 1 to 6 carbon atoms and aliphatic alcohols having 1 to 6 carbon atoms without any olefin to obtain the catalyst for use in producing a lower aliphatic carboxylic acid ester.

4. The process as claimed in claim 3, wherein the second step is performed in a reactor which is used in reacting an olefin selected from the group consisting of ethylene, propylene, n-butene, isobutene and a mixture of two or more thereof with an aliphatic carboxylic acid having 1 to 4 carbon atoms in the gas phase.

5. The process as claimed in claim 3 or 4, wherein the heteropolyacid salt is at least one salt selected from the group consisting of lithium salts, sodium salts, magnesium salts, barium salts, copper salts, gold salts and gallium salts of silicotungstic acid, phosphotungstic acid, phospho-molybdic acid, silicomolybdic acid, silicovanadotungstic acid, phosphovanadotungstic acid and phosphovanadomolybdic acid.

6. The process as claimed in any one of claims 3 to 5, wherein the second step is performed at a temperature of 80 to 300°C.

7. The process as claimed in any one of claims 3 to 6, wherein the second step is performed under a pressure of 0 to 3 MPaG (gauge pressure).

8. The process as claimed in any one of claims 3 to 7, wherein the second step is performed at a gas hourly space velocity (GHSV) of 100 to 7,000 hr⁻¹.

9. The process as claimed in any one of claims 3 to 8, wherein the gas containing at least one member selected from the group consisting of water, aliphatic carboxylic acids having 1 to 6 carbon atoms and aliphatic alcohols having 1 to 6 carbon atoms used in the second step is a mixed gas of water and acetic acid.

10. A process for producing a lower aliphatic carboxylic acid ester, comprising reacting an olefin selected from the group consisting of ethylene, propylene, n-butene, isobutene and a mixture of two or more thereof with an aliphatic carboxylic acid having 1 to 4 carbon atoms in the gas phase in the presence of the catalyst as claimed in any one of claims 1 and 2.

11. A process according to claim 10, wherein the reaction system additionally contains water.

12. A process for producing a lower aliphatic carboxylic acid ester, comprising reacting an olefin selected from the group consisting of ethylene, propylene, n-butene, isobutene and a mixture of two or more thereof, with an aliphatic carboxylic acid having 1 to 4 carbon atoms in the gas phase, which process comprises the following first to fourth steps:
First Step
a step of loading one or more heteropolyacid salts on a support to obtain a heteropolyacid salt supported catalyst;
Second Step
a step of filling the heteropolyacid salt supported catalyst obtained in the first step, into a reactor for use in the reaction of the olefin selected from the group consisting of ethylene, propylene, n-butene, isobutene and a mixture of two or more thereof with the aliphatic carboxylic acid having 1 to 4 carbon atoms in the gas phase;
Third Step
a step of contacting the heteropolyacid salt supported catalyst filled in the reactor, with a gas containing at least one member selected from the group consisting of water, aliphatic carboxylic acids having 1 to 6 carbon atoms and aliphatic alcohols having 1 to 6 carbon atoms without any olefin; and, distinguished from the Third Step.
as a Fourth Step
a step of passing a mixed gas containing the olefin selected from the group consisting of ethylene, propylene, n-butene, isobutene and a mixture of two or more thereof and the aliphatic carboxylic acid having 1 to 4 carbon atoms through the heteropolyacid salt supported catalyst after the third step, to obtain the lower aliphatic carboxylic acid ester.

13. The process as claimed in claim 12, wherein the heteropolyacid salt is at least one salt selected from the group consisting of lithium salts, sodium salts, magnesium salts, barium salts, copper salts, gold salts and gallium salts of silicotungstic acid, phosphotungstic acid, phosphomolybdic acid, silicomolybdic acid, silicovanadotungstic acid, phosphovanadotungstic-acid and phosphovanadomolybdic acid.

14. The process as claimed in claim 12 or 13, wherein the third step is performed at a temperature of 80 to 300° C.

15. The process as claimed in any one of claims 12 to 14, wherein the third step is performed under a pressure of 0 to 3 MPaG (gauge pressure).

16. The process as claimed in any one of claims 12 to 15, wherein the third step is performed at a gaseous hourly space velocity (GHSV) of 100 to 7,000 hr⁻¹.

17. The process as claimed in any one of claims 12 to 16, wherein the aliphatic carboxylic acid having 1 to 6 carbon atoms used in the third step is the same as the aliphatic carboxylic acid having 1 to 4 carbon atoms used in producing the lower aliphatic carboxylic acid ester.

18. The process as claimed in any one of claims 12 to 17, wherein the mixed gas containing an olefin selected from the group consisting of ethylene, propylene n-butene, isobutene and a mixture of two or more thereof and an aliphatic carboxylic acid having 1 to 4 carbon atoms used in the fourth step contains water.

## Patentansprüche

1. Katalysator zur Verwendung bei der Herstellung eines niederen aliphatischen Carbonsäureesters, der bei der Umsetzung eines Olefins, ausgewählt aus der Gruppe bestehend aus Ethylen, Propylen, n-Buten, Isobuten und einem Gemisch zweier oder mehrerer hiervon, mit einer aliphatischen Carbonsäure mit 1 bis 4 Kohlenstoffatomen in der Gasphase verwendbar ist, um einen niederen aliphatischen Carbonsäureester herzustellen, wobei der Katalysator durch ein Verfahren erhältlich ist, welches die folgenden ersten und zweiten Schritte umfasst:
Erster Schritt
einen Schritt des Aufbringens eines oder mehrerer Heteropolysäuresalze auf einen Träger, um einen Trägerkatalysator mit Heteropolysäuresalz zu erhalten; und
Zweiter Schritt
einen Schritt des Kontaktierens des in Schritt 1 erhaltenen Trägerkatalysators mit Heteropolysäuresalz mit einem Gas, das mindestens ein Mitglied enthält, ausgewählt aus der Gruppe, bestehend aus Wasser, aliphatischen Carbonsäuren mit 1 bis 6 Kohlenstoffatomen und aliphatischen Alkoholen mit 1 bis 6 Kohlenstoffatomen ohne ein Olefin, um den Katalysator zur Verwendung bei der Herstellung eines niederen aliphatischen Carbonsäureesters zu erhalten.

2. Katalysator nach Anspruch 1, wobei das Heteropolysäuresalz mindestens ein Salz ist, das aus der Gruppe ausgewählt ist, die aus Lithiumsalzen, Natriumsalzen, Magnesiumsalzen, Bariumsalzen, Kupfersalzen, Goldsalzen und Galliumsalzen von Siliciumwolframsäure, Phosphorwolframsäure, Phospormolybdänsäure, Siliciummolybdänsäure, Siliciumvanadiumwolframsäure, Phosphorvanadiumwolframsäure und Phosphorvanadiummolybdänsäure besteht.

3. Verfahren zur Herstellung eines Katalysators zur Verwendung bei der Herstellung eines niederen aliphatischen Carbonsäureesters, wobei der Katalysator bei der Umsetzung eines Olefins, ausgewählt aus der Gruppe bestehend aus einem Ethylen, Propylen, n-Buten, Isobuten und einem Gemisch zweier oder mehrerer hiervon, mit einer aliphatischen Carbonsäure mit 1 bis 4 Kohlenstoffatomen in der Gasphase verwendbar ist, um den niederen aliphatischen Carbonsäureester herzustellen, wobei das Verfahren die folgenden ersten und zweite Schritte umfasst:
Erster Schritt
einen Schritt des Aufbringens eines oder mehrerer Heteropolysäuresalze auf einen Träger, um einen Trägerkatalysator mit Heteropolysäuresalz zu erhalten; und
Zweiter Schritt
einen Schritt des Kontaktierens des in Schritt 1 erhaltenen Trägerkatalysators mit Heteropolysäuresalz mit einem Gas, das mindestens ein Mitglied enthält, ausgewählt aus der Gruppe, bestehend aus Wasser, aliphatischen Carbonsäuren mit 1 bis 6 Kohlenstoffatomen und aliphatischen Alkoholen mit 1 bis 6 Kohlenstoffatomen ohne ein Olefin, um den Katalysator zur Verwendung bei der Herstellung eines niederen aliphatischen Carbonsäureesters zu erhalten.

4. Verfahren nach Anspruch 3, wobei der zweite Schritt in einem Reaktionsraum durchgeführt wird, der bei der Umsetzung eines Olefins, das aus der Gruppe ausgewählt ist, bestehend aus Ethylen, Propylen, n-Buten, Isobuten und einem Gemisch zweier oder mehrerer hiervon, mit einer aliphatischen Carbonsäure mit 1 bis 4 Kohlenstoffatomen in der Gasphase verwendet wird.

5. Verfahren nach Anspruch 3 oder 4, wobei das Heteropolysäuresalz mindestens ein Salz ist, das aus der Gruppe ausgewählt ist, die aus Lithiumsalzen, Natriumsalzen, Magnesiumsalzen, Bariumsalzen, Kupfersalzen, Goldsalzen und Galliumsalzen von Siliciumwolframsäure, Phosphorwolframsäure, Phospormolybdänsäure, Siliciummolybdänsäure, Siliciumvanadiumwolframsäure, Phosphorvanadiumwolframsäure und Phosphorvanadiummolybdänsäure besteht.

6. Verfahren nach einem der Ansprüche 3 bis 5, wobei der zweite Schritt bei einer Temperatur von 80 bis 300 °C durchgeführt wird.

7. Verfahren nach einem der Ansprüche 3 bis 6, wobei der zweite Schritt unter einem Druck von 0 bis 3 MPaG (angezeigter Druck) durchgeführt wird.

8. Verfahren nach einem der Ansprüche 3 bis 7, wobei der zweite Schritt bei einer stündlichen Gasraumgeschwindigkeit (GHSV) von 100 bis 7.000 h⁻¹ durchgeführt wird.

9. Verfahren nach einem der Ansprüche 3 bis 8, wobei das in Schritt 2 verwendete Gas, welches mindestens ein Mitglied enthält, ausgewählt aus der Gruppe, bestehend aus Wasser, aliphatischen Carbonsäuren mit 1 bis 6 Kohlenstoffatomen und aliphatischen Alkoholen mit 1 bis 6 Kohlenstoffatomen, ein gemischtes Gas aus Wasser und Essigsäure ist.

10. Verfahren zur Herstellung eines niederen aliphatischen Carbonsäuresters, umfassend das Umsetzen eines Olefins, ausgewählt aus der Gruppe, bestehend aus Ethylen, Propylen, n-Buten, Isobuten und einem Gemisch zweier oder mehrerer hiervon, mit einer aliphatischen Carbonsäure mit 1 bis 4 Kohlenstoffatomen in der Gasphase in Gegenwart eines Katalysators nach einem der Ansprüche 1 und 2.

11. Verfahren nach Anspruch 10, wobei das Reaktionssystem zusätzlich Wasser enthält.

12. Verfahren zur Herstellung eines niederen aliphatischen Carbonsäureesters, umfassend das Umsetzen eines Olefins, ausgewählt aus der Gruppen, bestehend aus Ethylen, Propylen, n-Buten, Isobuten und einem Gemisch zweier oder mehrerer hiervon, mit einer aliphatischen Carbonsäure mit 1 bis 4 Kohlenstoffatomen in der Gasphase, wobei das Verfahren die folgenden ersten bis vierten Schritte umfasst:
Erster Schritt
einen Schritt des Aufbringens eines oder mehrerer Heteropolysäuresalze auf einen Träger, um einen Trägerkatalysator mit Heteropolysäuresalz zu erhalten; und
Zweiter Schritt
einen Schritt des Einfüllens des in dem ersten Schritt erhaltenen Trägerkatalysators mit Heteropolysäuresalz in einen Reaktionsraum zur Verwendung bei der Umsetzung des Olefins, das aus der Gruppe ausgewählt ist, bestehend aus Ethylen, Propylen, n-Buten, Isobuten und einem Gemisch zweier oder mehrerer hiervon, mit der aliphatischen Carbonsäure mit 1 bis 4 Kohlenstoffatomen in der Gasphase;
Dritter Schritt
einen Schritt des Kontaktierens des in den Reaktionsraum gefüllten Trägerkatalysators mit Heteropolysäuresalz mit einem Gas, das mindestens ein Mitglied enthält, ausgewählt aus der Gruppe, bestehend aus Wasser, aliphatischen Carbonsäuren mit 1 bis 6 Kohlenstoffatomen und aliphatischen Alkoholen mit 1 bis 6 Kohlenstoffatomen ohne ein Olefin; und, im Unterschied zum dritten Schritt,
als Vierten Schritt
einen Schritt des Durchströmens eines gemischten Gases, enthaltend das Olefin, ausgewählt aus der Gruppe, bestehend aus Ethylen, Propylen, n-Buten, Isobuten,und einem Gemisch zweier oder mehrerer hiervon, und die aliphatische Carbonsäure mit 1 bis 4 Kohlenstoffatomen, durch den Trägerkatalysator mit Heteropolysäuresalz nach dem dritten Schritt, um den niederen aliphatischen Carbonsäureester zu erhalten.

13. Verfahren nach Anspruch 12, wobei das Heteropolysäuresalz mindestens ein Salz ist, das aus der Gruppe ausgewählt ist, die aus Lithiumsalzen, Natriumsalzen, Magnesiumsalzen, Bariumsalzen, Kupfersalzen, Goldsalzen und Galliumsalzen von Siliciumwolframsäure, Phosphorwolframsäure, Phospormolybdänsäure, Siliciummolybdänsäure, Siliciumvanadiumwolframsäure, Phosphorvandiumwolframsäure und Phosphorvanadiummolybdänsäure besteht.

14. Verfahren nach Anspruch 12 oder 13, wobei der dritte Schritt bei einer Temperatur von 80 bis 300 °C durchgeführt wird.

15. Verfahren nach einem der Ansprüche 12 bis 14, wobei der dritte Schritt bei einem Druck von 0 bis 3 MPaG (angezeigter Druck) durchgeführt wird.

16. Verfahren nach einem der Ansprüche 12 bis 15, wobei der dritte Schritt bei einer stündlichen Gasraumgeschwindigkeit (GHSV) von 100 bis 7.000 h⁻¹ durchgeführt wird.

17. Verfahren nach einem der Ansprüche 12 bis 16, wobei die in dem dritten Schritt verwendete aliphatische Carbonsäure mit 1 bis 6 Kohlenstoffatomen die gleiche ist wie die aliphatische Carbonsäure mit 1 bis 4 Kohlenstoffatomen, die bei der Herstellung des niederen aliphatischen Carbonsäureesters verwendet wird.

18. Verfahren nach einem der Ansprüche 12 bis 17, wobei das in dem vierten Schritt verwendete gemischte Gas, welches ein Olefin enthält, das aus der Gruppe ausgewählt ist, bestehend aus Ethylen, Propylen, n-Buten, Isobuten und einem Gemisch zweier oder mehrerer hiervon, sowie eine aliphatische Carbonsäure mit 1 bis 4 Kohlenstoffatomen, Wasser enthält.

## Revendications

1. Catalyseur utile dans la production d'un ester d'acide carboxylique aliphatique inférieur, qui est utilisé dans la réaction d'une oléfine choisie dans le groupe constitué par l'éthylène, le propylène, le n-butène, l'isobutène et un mélange de deux ou plusieurs d'entre eux avec un acide carboxylique aliphatique ayant 1 à 4 atomes de carbone en phase gazeuse pour produire un ester d'acide carboxylique aliphatique inférieur, ce catalyseur pouvant être obtenu par un procédé comprenant les première et seconde étapes suivantes :
Première étape
Etape pour le chargement d'un ou plusieurs sels d'hétéropolyacides sur un support pour obtenir un catalyseur supporté à base de sel d'hétéropolyacide ; et
Seconde étape
Etape pour la mise en contact du catalyseur supporté à base de sel d'hétéropolyacide obtenu dans la première étape avec un gaz contenant au moins un élément choisi dans le groupe constitué par l'eau, les acides carboxyliques aliphatiques ayant 1 à 6 atomes de carbone et les alcools aliphatiques ayant 1 à 6 atomes de carbone sans aucune oléfine pour obtenir le catalyseur utile dans la production d'un ester d'acide carboxylique aliphatique inférieur.

2. Catalyseur selon la revendication 1, où le sel d'hétéropolyacide est au moins un sel choisi dans le groupe constitué par les sels de lithium, les sels de sodium, les sels de magnésium, les sels de baryum, les sels de cuivre, les sels d'or et les sels de gallium de l'acide silicotungstique, l'acide phosphotungstique, l'acide phosphomolybdique, l'acide silicomolybdique, l'acide silicovanadotungstique, l'acide phosphovanadotungstique et l'acide phosphovanadomolybdique.

3. Procédé pour la production d'un catalyseur utile pour la production d'un ester d'acide carboxylique aliphatique inférieur, ledit catalyseur étant utilisé dans la réaction d'une oléfine choisie dans le groupe constitué par l'éthylène, le propylène, le n-butène, l'isobutène et un mélange de deux ou plusieurs d'entre eux avec un acide carboxylique aliphatique ayant 1 à 4 atomes de carbone en phase gazeuse pour produire un ester d'acide carboxylique aliphatique inférieur, ledit procédé comprenant les première et seconde étapes suivantes :
Première étape
Etape pour le chargement d'un ou plusieurs sels d'hétéropolyacides sur un support pour obtenir un catalyseur supporté à base de sel d'hétéropolyacide ; et
Seconde étape
Etape pour la mise en contact du catalyseur supporté à base de
sel d'hétéropolyacide obtenu dans la première étape avec un gaz contenant au moins un élément choisi dans le groupe constitué par l'eau, les acides carboxyliques aliphatiques ayant 1 à 6 atomes de carbone et les alcools aliphatiques ayant 1 à 6 atomes de carbone sans aucune oléfine pour obtenir un ester d'acide carboxylique aliphatique inférieur.

4. Procédé selon la revendication 3, dans lequel la seconde étape est réalisée dans un réacteur utilisé dans la réaction d'une oléfine choisie dans le groupe constitué par l'éthylène, le propylène, le n-butène, l'isobutène et un mélange de deux ou plusieurs d'entre eux avec un acide carboxylique aliphatique ayant 1 à 4 atomes de carbone en phase gazeuse.

5. Procédé selon la revendication 3 ou 4, où le sel d'hétéropolyacide est au moins un sel choisi dans le groupe constitué par les sels de lithium, les sels de sodium, les sels de magnésium, les sels de baryum, les sels de cuivre, les sels d'or et les sels de gallium de l'acide silicotungstique, l'acide phosphotungstique, l'acide phosphomolybdique, l'acide silicomolybdique, l'acide silicovanadotungstique, l'acide phosphovanadotungstique et l'acide phosphovanadomolybdique.

6. Procédé selon l'une quelconque des revendications 3 à 5, où la seconde étape est réalisée à une température de 80 à 300°C.

7. Procédé selon l'une quelconque des revendications 3 à 6, où la seconde étape est réalisée sous une pression de 0 à 3 MPaG (pression manométrique).

8. Procédé selon l'une quelconque des revendications 3 à 7, où la seconde étape est réalisée à une vitesse spatiale horaire gazeuse (GHSV) de 100 à 7000 heure⁻¹.

9. Procédé selon l'une quelconque des revendications 3 à 8, dans lequel le gaz contenant au moins un élément choisi dans le groupe constitué par l'eau, les acides carboxyliques aliphatiques ayant 1 à 6 atomes de carbone et les alcools aliphatiques ayant 1 à 6 atomes de carbone utilisé dans la seconde étape est un gaz mixte d'eau et d'acide acétique.

10. Procédé pour la production d'un ester d'acide carboxylique aliphatique inférieur, comprenant la réaction d'une oléfine choisie dans le groupe constitué par l'éthylène, le propylène, le n-butène, l'isobutène et un mélange de deux ou plusieurs d'entre eux avec un acide carboxylique aliphatique ayant 1 à 4 atomes de carbone en phase gazeuse en présence du catalyseur selon l'une quelconque des revendications 1 et 2.

11. Procédé selon la revendication 10, où le système réactionnel contient en outre de l'eau.

12. Procédé pour la production d'un ester d'acide carboxylique aliphatique inférieur, comprenant la réaction d'une oléfine choisie dans le groupe constitué par l'éthylène, le propylène, le n-butène, l'isobutène et un mélange de deux ou plusieurs d'entre eux avec un acide carboxylique aliphatique ayant 1 à 4 atomes de carbone en phase gazeuse, ce procédé comprenant les étapes un à quatre suivantes :
Première étape
Etape de chargement d'un ou plusieurs sels d'hétéropolyacides sur un support pour obtenir un catalyseur supporté à base de sel d'hétéropolyacide ;
Seconde étape
Etape d'introduction du catalyseur supporté à base de sel d'hétéropolyacide obtenu dans la première étape dans un réacteur pour l'utiliser dans la réaction de l'oléfine choisie dans le groupe constitué par l'éthylène, le propylène, le n-butène, l'isobutène et un mélange de deux ou plusieurs d'entre eux avec l'acide carboxylique aliphatique ayant 1 à 4 atomes de carbone en phase gazeuse ;
Troisième étape
Etape de mise en contact du catalyseur supporté à base de sel d'hétéropolyacide introduit dans le réacteur, avec un gaz contenant au moins un élément choisi dans le groupe constitué par l'eau, les acides carboxyliques aliphatiques ayant 1 à 6 atomes de carbone et les alcools aliphatiques ayant 1 à 6 atomes de carbone sans aucune oléfine ; et, séparément de la troisième étape,
En quatrième étape
Etape de passage d'un gaz mixte contenant l'oléfine, choisie dans le groupe constitué par l'éthylène, le propylène, le n-butène, l'isobutène et un mélange de deux ou plusieurs d'entre eux, et l'acide carboxylique aliphatique ayant 1 à 4 atomes de carbone dans le catalyseur supporté à base de sel d'hétéropolyacide après la troisième étape, pour obtenir l'ester d'acide carboxylique aliphatique inférieur.

13. Procédé selon la revendication 12, où le sel d'hétéropolyacide est au moins un sel choisi dans le groupe constitué par les sels de lithium, les sels de sodium, les sels de magnésium, les sels de baryum, les sels de cuivre, les sels d'or et les sels de gallium de l'acide silicotungstique, l'acide phosphotungstique, l'acide phosphomolybdique, l'acide silicomolybdique, l'acide silicovanadotungstique, l'acide phosphovanadotungstique et l'acide phosphovanadomolybdique.

14. Procédé selon la revendication 12 ou 13, où la troisième étape est réalisée à une température de 80 à 300°C.

15. Procédé selon l'une quelconque des revendications 12 à 14, où la troisième étape est réalisée sous une pression de 0 à 3 MPaG (pression manométrique).

16. Procédé selon l'une quelconque des revendications 12 à 15, où la troisième étape est réalisée à une vitesse spatiale horaire gazeuse (GHSV) de 100 à 7000 heure⁻¹.

17. Procédé selon l'une quelconque des revendications 12 à 16, où l'acide carboxylique aliphatique ayant 1 à 6 atomes de carbone dans la troisième étape est le même que l'acide carboxylique aliphatique ayant 1 à 4 atomes de carbone utilisé dans la production de l'ester d'acide carboxylique aliphatique inférieur.

18. Procédé selon l'une quelconque des revendications 12 à 17, où le gaz mixte, contenant une oléfine, choisie dans le groupe constitué par l'éthylène, le propylène, le n-butène, l'isobutène et un mélange de deux ou plusieurs d'entre eux, et un acide carboxylique aliphatique ayant 1 à 4 atomes de carbone, utilisé dans la quatrième étape contient de l'eau.
